# EUROPÄISCHE PATENTSCHRIFT

(11) **EP 2 159 565 B1**
(45) Veröffentlichungstag und Bekanntmachung des Hinweises auf die Patenterteilung: **18.02.2015**
(21) Anmeldenummer: 09010109.8
(22) Anmeldetag: 05.08.2009
(51) Int. Cl.: G01N 21/03, G01N 33/49

(54) **Einmal-Mikroküvette zur Bestimmung des Hämoglobingehalts in Vollblut**
One-off micro-cuvette for determining the haemoglobin content in whole blood
Micro-cuvette à usage unique destinée à la détermination du taux d'hémoglobine dans le sang total

(30) Priorität: 25.08.2008 DE 102008039810
(43) Veröffentlichungstag der Anmeldung: 03.03.2010
(73) Patentinhaber: EKF - diagnostic GmbH, 39179 Barleben (DE)
(72) Erfinder: Dumschat, Christa, Dr., 39179 Barleben (DE); Hensel, Sike, 39108 Magdeburg (DE); Hirschfelder, Monika, Dr., 39179 Barleben (DE)
(74) Vertreter: Lins, Edgar

(56) Entgegenhaltungen:
- DE-A1-102006 025 477
- DE-T2- 69 026 396

## Beschreibung

Die Erfindung betrifft eine Einmal-Mikroküvette zur Bestimmung des Hämoglobingehalts in Vollblut ohne chemische Umsetzung des Hämoglobins, mit einer spaltförmigen Kavität, die zum Einziehen des Vollbluts in die Kavität der Küvette durch eine Kapillarkraft ausgebildet und wenigstens in einem Teilbereich durch zwei im Wesentlichen parallele Küvettenwände begrenzt ist, die auf ihrer zur Kavität gerichteten Oberfläche wenigstens teilweise mit einem trockenen Hämolysemittel beschichtet sind, wobei des Hämolysemittel durch das Vollblut lösbar und zur Hämolyse von Blutzellen geeignet ist und wobei die Küvettenwände wenigstens in Teilbereichen zu Messzwecken durchstrahlbar sind.

Eine Einmal-Mikroküvette zur Hämoglobinbestimmung ist beispielsweise durch die US 5,674,457 bekannt. Die Mikroküvette wird vorzugsweise als einstückiges Spritzgussteil aus einem geeigneten, durchsichtigen Kunststoff hergestellt, der in den durchstrahlbaren Teilbereichen der Küvettenwände optisch poliert ist, um eine störungsfreie Messung einer Farbreaktion durchführen zu können. Die Innenwände der Kavität sind mit einem trockenen Reagens beschichtet, das aus Natriumdesoxycholat, Natriumazid und Natriumnitrit besteht. Zur Bestimmung von Hämoglobin in einer Vollblutprobe wird somit die Azidmethämoglobinmethode verwendet, bei der drei Reagenzien eingesetzt werden. Als Hämolysemittel wird beispielsweise Natriumdesoxycholat verwendet, das die Zellwände der roten Blutkörperchen an- bzw. auflöst. Das zuvor in den Erythrocyten eingeschlossene Hämoglobin liegt somit als freie Lösung vor.

Durch ein weiteres Reagens, Natriumnitrit (NaN02) wird das zweiwertige Eisen des Oxyhämoglobins und des Desoxyhämoglobins zu dreiwertigem Eisen im Methämoglobin oxdiert. Azidionen aus dem dritten Reagens, Natriumazid (NaN3) bilden mit dem Methämoglobin einen farbigen Komplex, der Absorptionsmaxima bei 540 und 575 nm aufweist und somit fotometrisch quantitativ bestimmt werden kann. Es wird zusätzliche eine Kontrollmessung bei einer Wellenlänge durchgeführt, bei der keine der relevanten Hämoglobinformen eine merkbare Absorption aufweist. Die Kontrollmessung dient daher der Kompensation von Trübungen des optischen Strahlengangs und wird beispielsweise bei einer Wellenlänge von 880 nm durchgeführt.

Die Einmal-Mikroküvetten dienen einer schnellen und kostengünstigen Bestimmung des Hämoglobingehalts, beispielsweise für ein Blutspenderscreening. Um einen effektiven Ablauf zu erreichen, ist eine möglichst kurze Messzeit des Hämoglobinmessgeräts wünschenswert.

Es ist ferner bekannt, eine chemische oder mechanische (durch Ultraschall) Hämolyse der Blutprobe vorzunehmen, ohne eine anschließende chemische Umsetzung des Hämoglobins, beispielsweise in Azidmethämoglobin, vorzunehmen. Es wird dann bei bestimmten Wellenlängen die Absorption gemessen und durch eine Matrixkalkulation die gesamte Hämoglobinkonzentration und die der Hämoglobinderivate bestimmt. Derartige Geräte sind für die klinische Analyse, beispielsweise in Intensivstationen, üblich. In ähnlicher Weise ist in der WO02/01195A1 offenbart, lediglich die Hämolyse mittels eines Hämolysemittels, vorzugsweise Natriumdesoxycholat, Kaliumdesoxycholat oder eine Mischung hiervon, vorzunehmen, ohne eine anschließende Reaktion mit einem Nitrit und/oder einem Azid durchzuführen. Diese zusätzlichen Reagenzien werden als hygroskopisch angesehen und für eine mangelnde Lagerfähigkeit der Mikroküvetten verantwortlich gemacht. Aufgrund der fehlenden chemischen Umsetzung der Hämoglobinformen wird eine Absorptionsmessung bei 490 bis 520 nm durchgeführt. Die Kompensationsmessung für die Berücksichtigung der Trübung findet im üblichen Bereich zwischen 850 und 910 nm statt. Mit diesen Verfahren wird aufgrund der nicht mehr benötigten chemischen Umsetzungen des hämolysierten Blutes eine gewisse Reaktionszeit eingespart.

Das Einziehen des Vollblutes in die Kavität der Küvette durch eine Kapillarkraft ist jedoch problematisch. Bei der Verwendung von Desoxycholat als Hämolysierungsmittel findet ein stark verzögertes Einziehen des Bluttropfens in die Kavität der Küvette statt. In vielen Fällen gelingt das Einziehen überhaupt nicht oder geht mit einer störenden Luftbläschenbildung einher. Die angestrebte Beschleunigung des Messvorgangs lässt sich daher auf diese Weise in der Praxis nicht erreichen.

DE 10 2006 025 477 A1 offenbart eine Einmal-Mikroküvette die dazu dient, einen Blutstropfen durch Einziehen des Blutstropfens in die Kavität der Mikroküvette aufzunehmen. Durch das Reagens auf den zueinander parallelen Küvettenwänden, die die Kavität begrenzen (also auf den Innenwänden), wird das Blut hämolysiert, also muss durch das Vollblut lösbar sein. Demzufolge ist bereits das Hämolysemittel, also das auf die Innenwände der Küvette aufgebrachte Reagens, ausreichend hydrophil, um in dem bekanntlich wässrigen Blut gelöst zu werden.

Die vorliegende Erfindung geht von der Problemstellung aus, eine Beschleunigung des Ablaufes vom Einziehen des Bluttropfens bis zur aussagekräftigen Durchführung der Messung zu erzielen.

Zur Lösung dieser Aufgabe ist bei einer Einmal-Mikroküvette der eingangs erwähnten Art erfindungsgemäß vorgesehen, dass mit dem Hämolysemittel wenigstens ein Additiv auf den Wänden abgeschieden ist und dass wenigstens ein Additiv die Form eines inerten Salzes oder einer Mischung von inerten Salzen, womit das Vollblut besser und schneller in die Kavität einziehbar ist, aufweist.

Während die bisherigen Ansätze zur Beschleunigung des Messverfahrens auf die chemische Umsetzung des Blutes mit geeigneten Reagenzien gerichtet waren, wird mit der vorliegenden Erfindung eine Beschleunigung des Messvorganges durch die Beschleunigung physikalischer Vorgänge erzielt. Die Zugabe eines inerten, d. h. bezüglich des Hämoglobins nicht reaktiven Salzes führt in überraschender Weise dazu, dass das Vollblut wesentlich besser und schneller in die spaltförmige Kavität der Einmal-Mikroküvette durch Kapillareffekte eingezogen wird und darüber hinaus dass sich das Hämolysemittel schneller in dem Vollblut auflöst, sodass die hämolysierende Wirkung auf die Blutzellen beschleunigt wird. Von besonderer Bedeutung ist dabei die Verbesserung des Einzugsverhaltens für den Blutstropfen in die spaltförmige Kavität der Mikroküvette, da hierbei eine Beschleunigung um wenigstens den Faktor 2 erreicht wird.

Der erfindungsgemäße Effekt des beschleunigten Einziehens des Bluttropfens in die spaltförmige Kavität wird noch dadurch verbessert, dass auf die Innenseiten der Küvettenwände als weiteres Additiv ein nicht reaktives wasserlösliches Polymer abgeschieden wird. In Kombination mit dem inerten Salz wird hierdurch eine weitere Beschleunigung des Einzugverhaltens erreicht.

Ein ähnlicher beschleunigender Effekt wird - auch kumulativ - dadurch erreicht, dass als ein weiteres Additiv auf den Innenseiten der Küvettenwände ein Anticoagulanz abgeschieden wird. Da das Anticoagulanz zu einer Beschleunigung des unter Kapillarwirkung in die spaltförmige Kavität eingezogenen Blutstropfens führt, ist davon auszugehen, dass die Störung des Einziehens des Blutstropfens bei den bisherigen Mikroküvetten auch von Verklumpungsvorgängen und Clusterbildungen im Blut gestört wird.

Schließlich ist erfindungsgemäß vorgesehen, dass zu dem Hämolysemittel als weiteres Additiv ein Farbstoff auf den Wänden abgeschieden wird. Hierdurch ist eine Qualitätskontrolle für die auf den Innenwänden aufgebrachte Beschichtung möglich, die aufgrund der Farbstoffzugabe sichtbar wird und auf diese Weise auf Gleichmäßigkeit überprüft werden kann. Die gleichmäßige Aufbringung der Beschichtung - zumindest im Messbereich der Küvette - stellt nämlich ein wesentliches Kriterium für die Genauigkeit der Messung dar und ist für das Erzielen eines luftbläschenfreien Einziehens des Bluttropfens von entscheidender Bedeutung.

Durch die Verwendung der genannten Additive gelingt es somit, eine einen Bluttropfen schnell und störungsfrei (luftbläschenfrei) einziehende Mikroküvette zu erstellen, die eine schnellere Messung als bisher ermöglicht.

Der Küvettenkörper der Mikroküvette wird vorzugsweise einstückig im Spritzgießverfahren hergestellt. In diesem Fall wird die Beschichtung auf der Innenseite der Mikroküvette dadurch hergestellt, dass das Hämolysemittel und mit ihm das wenigstens eine Additiv als Lösung in die Kavität eingefüllt wird und das Lösungsmittel anschließend verdampfen kann. Demgemäß muss das Hämolysemittel durch ein geeignetes, leicht flüchtiges Lösungsmittel lösbar sein, beispielsweise in Alkohol. Geeignete Hämolysemittel sind bekannt und beispielsweise in der WO02/01195A1 aufgezählt. Bevorzugt ist die Verwendung von Desoxycholaten, wie Natrium-, Kalium-, Calcium-, Morpholin- und Ammoniumdesoxycholat. Bevorzugt wird Natriumdesoxycholat verwendet. Der Anteil des Hämolysemittels in der Beschichtung beträgt vorzugsweise 70 bis 99,9 Gew.-%. Bei der bevorzugten Verwendung mehrerer Additive ist der Anteil des Hämolysemittels von 70 bis 85 Gew.-% bevorzugt.

Das erfindungsgemäß zugegebene inerte Salz, das auch durch eine Mischung mehrerer Salze gebildet sein kann, ist beispielsweise Natriumchlorid oder Kaliumchlorid. Der Anteil des inerten Salzes in der Beschichtung beträgt 0,1 bis 20 Gew.-%, bevorzugt 0,2 bis 10 Gew.-%, weiter bevorzugt 0,5 bis 2 Gew.-%.

Beispiele für erfindungsgemäß als Additiv vorgesehene nichtreaktive wasserlösliche Polymere sind Polyethylenglycol, Polypropylenglycol, Blockcopolymere aus Ethylenoxid und Propylenoxid, funktionalisierte Silikone, Polyvinylpyrrolidone, Polyvinylalkohole, Polyvinylacetate, Polyalkylenglycolether (Handelsname Brij), Polyethylensorbitanmonolaurat, Polyethylenglycolsorbitanmonostearat, Polyethylenglycolsorbitanoleat (Handelsname Tween), Octylglycosid, Diethylenglykol, Ethylenglykol-diglycidylether, Glycerol. Der Anteil der nichtreaktiven wasserlöslichen Polymere in der Beschichtung beträgt vorzugsweise 0,5 bis 25 Gew.-%, bevorzugt 12 bis 24 Gew.-%, weiter bevorzugt 15 bis 20 Gew.-%.

Beispiele für geeignete Antikoagulanzien als weiteres Additiv sind Heparin und Heparinsalze, wie Heparin-Natrium, Heparin-Kalium, Heparin-Ammonium und Heparin-Lithium, Citrat, Natriumfluorid, ACD (Acid-Citrat-Dextrose), Jodacetat, Hirudin, Oxalat, Acetylsalicylsäure und deren Salze, Warfarin, Coumarin und seine Derivate, Indan-1,3-dion, Pheprocoumaron, Dicumarol, Marcumar, Streptokinase, Urokinase, EDTA (Ethylendiamintetraessigsäure bzw. Ethylendiamintetraazetat).

Der Anteil des Antikoagulans als Additiv beträgt vorzugsweise 0,05 bis 5 Gew.-%, bevorzugt 1 bis 3 Gew.-%.

Der als weiteres Additiv vorgesehene Farbstoff kann beliebig gewählt werden, solange er die Hämolysereaktion nicht stört. Der Anteil des Farbstoffes in der Beschichtung beträgt vorzugsweise 0,2 bis 0,5 Gew.-%.

Die in der Mikroküvette verwendete Kavität hat im Messbereich vorzugsweise einen Abstand zwischen Innenseiten der Küvettenwände von 100 bis 200 µm. Außerhalb des Messbereichs, insbesondere im Bereich eines Einzugsspalts für den Bluttropfen beträgt der Abstand der Küvettenwände vorzugsweise zwischen 200 und 400 µm. Dadurch wird das Einziehen des Bluttropfens in die spaltförmige Kavität erleichtert. Innerhalb der Kavität entsteht somit ein stufenförmiger Übergang in den Messbereich, der eine geringere Küvettenhöhe, also einen geringeren Abstand zwischen den Innenseiten der Küvettenwände aufweist. Der Messbereich zeichnet sich üblicherweise durch die polierten Außenseiten der Küvettenwände aus, die das störungsfreie Durchstrahlen der Küvettenwände mit der in der Kavität enthaltenen Blutprobe begünstigen.

Als Material für den Küvettenkörper kommt jedes transparente Kunststoffmaterial in Frage. Bei der Herstellung eines einstückigen Küvettenkörpers im Spritzgießverfahren muss der Kunststoff naturgemäß spritzgussfähig sein.

Es ist auch möglich, die erfindungsgemäße Mikroküvette als wenigstens zweiteilige Küvette herzustellen, deren beide Teile in geeigneter Weise miteinander verbunden werden, beispielsweise durch Klebung. In diesem Fall wird das Aufbringen der Beschichtung erleichtert, da die Beschichtung auf die Innenseite wenigstens einer Küvettenwand in einem beliebigen Verfahren aufgebracht werden kann. Dabei ist es auch möglich, die Beschichtung nur auf einer Innenwand vorzusehen. Bevorzugt ist allerdings die Beschichtung beider gegenüberliegender Küvettenwände, zumindest im Messbereich der Mikroküvette, In der zweiteiligen Ausführungsform der Mikroküvette kann diese auch aus Glas oder anderen transparenten Materialien bestehen.

## Patentansprüche

1. Einmal-Mikroküvette zur Bestimmung des Hämoglobingehalts in Vollblut ohne chemische Umsetzung des Hämoglobins, mit einer spaltförmigen Kavität, die zum Einziehen des Vollbluts in die Kavität der Küvette durch eine Kapillarkraft ausgebildet und wenigstens in einem Teilbereich durch zwei im Wesentlichen parallele Küvettenwände begrenzt ist, die auf ihrer zur Kavität gerichteten Oberfläche wenigstens teilweise mit einem trockenen Hämolysemittel beschichtet sind, wobei des Hämolysemittel durch das Vollblut lösbar und zur Hämolyse von Blutzellen geeignet ist und wobei die Küvettenwände wenigstens in Teilbereichen zu Messzwecken durchstrahlbar sind, **dadurch gekennzeichnet, dass** mit dem Hämolysemittel zum beschleunigten Einziehen des Bluttropfens in die spaltförmige Kavität wenigstens ein Additiv auf den Wänden abgeschieden ist und dass wenigstens ein Additiv die Form eines inerten Salzes oder einer Mischung von inerten Salzen aufweist.

2. Einmal-Mikroküvette nach Anspruch 1, **dadurch gekennzeichnet, dass** das inerte Salz oder die Mischung von inerten Salzen einen Anteil von 0,1 bis 20 Gew.-%, der gesamten Beschichtung, gebildet durch das Hämolysemittel und das wenigstens eine Additiv, ausmacht.

3. Einmal-Mikroküvette nach Anspruch 1 oder 2, **dadurch gekennzeichnet, dass** als weiteres Additiv ein nichtreaktives wasserlösliches Polymer auf den Oberflächen der Küvettenwände abgeschieden ist.

4. Einmal-Mikroküvette nach Anspruch 3, **dadurch gekennzeichnet, dass** das Polymer einen Anteil von 0,5 bis 25 Gew.-% der Beschichtung ausmacht.

5. Einmal-Mikroküvette nach einem der Ansprüche 1 bis 4, **dadurch gekennzeichnet, dass** als weiteres Additiv ein Antikoagulans auf den Oberflächen der Küvettenwände abgeschieden ist.

6. Einmal-Mikroküvette nach Anspruch 5, **dadurch gekennzeichnet, dass** das Antikoagulans einen Anteil von 0,05 bis 5 Gew.-% der Beschichtung ausmacht.

7. Einmal-Mikroküvette nach einem der Ansprüche 1 bis 6, **dadurch gekennzeichnet, dass** als weiteres Additiv ein Farbstoff auf der Oberfläche der Küvettenwände abgeschieden ist.

8. Einmal-Mikroküvette nach Anspruch 7, **dadurch gekennzeichnet, dass** der Farbstoff einen Anteil von 0,2 bis 0,5 Gew.-% der Beschichtung ausmacht.

9. Einmal-Mikroküvette nach einem der Ansprüche 1 bis 8, **dadurch gekennzeichnet, dass** die Beschichtung durch Einbringen einer Lösung in die Kavität und Trocknung durch Ausdampfen des Lösungsmittels hergestellt ist,

## Claims

1. Disposable microcuvette for determining the haemoglobin content in whole blood without chemical reaction of the haemoglobin, having a slit-shaped cavity which is designed to draw the whole blood into the cavity of the cuvette by means of a capillary force and is bounded in at least a sub-region by two substantially parallel cuvette walls which are at least partly coated with a dry haemolysis agent on their surface directed towards the cavity, wherein the haemolysis agent is dissolvable by means of the whole blood and suited to haemolysis of blood cells and wherein the cuvette walls are penetrable at least in sub-regions by radiation for measurement purposes, **characterized in that** at least one additive is deposited on the walls with the haemolysis agent for quickened drawing-in of the blood drop into the slit-shaped cavity and **in that** at least one additive is in the form of an inert salt or a mixture of inert salts.

2. Disposable microcuvette according to Claim 1, **characterized in that** the inert salt or the mixture of inert salts makes up a proportion of from 0.1 to 20% by weight of the total coating formed by the haemolysis agent and the at least one additive.

3. Disposable microcuvette according to Claim 1 or 2, **characterized in that** a non-reactive water-soluble polymer is deposited on the surfaces of the cuvette walls as a further additive.

4. Disposable microcuvette according to Claim 3, **characterized in that** the polymer makes up a proportion of from 0.5 to 25% by weight of the coating.

5. Disposable microcuvette according to any of Claims 1 to 4, **characterized in that** an anticoagulant is deposited on the surfaces of the cuvette walls as a further additive.

6. Disposable microcuvette according to Claim 5, **characterized in that** the anticoagulant makes up a proportion of from 0.05 to 5% by weight of the coating.

7. Disposable microcuvette according to any of Claims 1 to 6, **characterized in that** a dye is deposited on the surface of the cuvette walls as a further additive.

8. Disposable microcuvette according to Claim 7, **characterized in that** the dye makes up a proportion of from 0.2 to 0.5% by weight of the coating.

9. Disposable microcuvette according to any of Claims 1 to 8, **characterized in that** the coating is obtained by introducing a solution into the cavity and drying by evaporation of the solvent.

## Revendications

1. Micro-cuvette à usage unique pour la détermination du taux d'hémoglobine dans du sang entier sans transformation chimique de l'hémoglobine, comprenant une cavité en forme de fente, qui est réalisée pour attirer le sang entier dans la cavité de la cuvette par une force capillaire et qui est délimitée au moins dans une zone partielle par deux parois de cuvette sensiblement paralléles, lesquelles sont revêtues au moins partiellement d'un agent d'hémolyse sec sur leurs surfaces tournées vers la cavité, dans laquelle l'agent d'hémolyse est soluble dans le sang entier et approprié pour l'hémolyse de cellules sanguines, et dans laquelle les parois de la cuvette sont susceptibles d'être traversées par un rayonnement au moins dans des zones partielles à des fins de mesure,
**caractérisée en ce que** au moins un additif est déposé sur les parois conjointement avec l'agent d'hémolyse pour attirer de façon accélérée la goutte de sang dans la cavité en forme de fente, et **en ce qu'**au moins un additif présente la forme d'un sel inerte ou d'un mélange de sels inertes.

2. Micro-cuvette à usage unique selon la revendication 1, **caractérisée en ce que** le sel inerte ou le mélange de sels inertes constitue une part de 0,1 à 20 % en poids du revêtement total, formé par l'agent d'hémolyse et par ledit au moins un additif.

3. Micro-cuvette à usage unique selon la revendication 1 ou 2, **caractérisée en ce que** l'on dépose à titre d'additif supplémentaire un polymère non réactif soluble dans l'eau sur les surfaces des parois de la cuvette.

4. Micro-cuvette à usage unique selon la revendication 3, **caractérisée en ce que** le polymère constitue une part de 0,5 à 25 % en poids du revêtement.

5. Micro-cuvette à usage unique selon l'une des revendications 1 à 4, **caractérisée en ce que** l'on dépose à titre d'additif supplémentaire un anticoagulant sur les surfaces des parois de la cuvette.

6. Micro-cuvette à usage unique selon la revendication 5, **caractérisée en ce que** l'anticoagulant constitue une part de 0,05 à 5% en poids du revêtement.

7. Micro-cuvette à usage unique selon l'une des revendications 1 à 6, **caractérisée en ce que** l'on dépose à titre d'additif supplémentaire un colorant sur la surface des parois de la cuvette.

8. Micro-cuvette à usage unique selon la revendication 7, **caractérisée en ce que** le colorant constitue une part de 0,2 à 0,5 % en poids du revêtement.

9. Micro-cuvette à usage unique selon l'une des revendications 1 à 8, **caractérisée en ce que** le revêtement est réalisé par introduction d'une solution dans la cavité et séchage par évaporation du solvant.
